(19) Europäisches Patentamt
European Patent Office
Office européen des brevets

(11) Veröffentlichungsnummer: **0 102 520**
B1

# (12) EUROPÄISCHE PATENTSCHRIFT

(45) Veröffentlichungstag der Patentschrift: **29.10.86**

(51) Int. Cl.[4]: **C 07 C 93/06,** C 07 C 121/75, C 07 D 263/14

(21) Anmeldenummer: **83107527.0**

(22) Anmeldetag: **30.07.83**

## (54) Verfahren zur Umkehrung der Konfiguration optisch aktiver Verbindungen und optisch aktive Zwischenprodukte dieses Verfahrens.

(30) Priorität: **04.08.82 DE 3229046**

(43) Veröffentlichungstag der Anmeldung: **14.03.84 Patentblatt 84/11**

(45) Bekanntmachung des Hinweises auf die Patenterteilung: **29.10.86 Patentblatt 86/44**

(84) Benannte Vertragsstaaten: **AT BE CH DE FR GB IT LI NL SE**

(56) Entgegenhaltungen: **EP - A - 0 007 605**

**CHEMICAL ABSTRACTS, vol. 84 (1976), nr. 43593x**

(73) Patentinhaber: **HOECHST AKTIENGESELLSCHAFT, Postfach 80 03 20, D-6230 Frankfurt am Main 80 (DE)**

(72) Erfinder: **Leuchs, Hans-Jürgen, Dr., Am Schellberg 42, D-6232 Bad Soden am Taunus (DE)**
Erfinder: **Mohler, Werner, Dr., Frankfurter Strasse 58, D-6238 Hofheim am Taunus (DE)**
Erfinder: **Erle, Hanns-Eberhard, Dr., Lauterbacher Strasse 38, D-6000 Frankfurt am Main 61 (DE)**

EP 0 102 520 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Umkehr der Konfiguration am optisch aktiven Kohlenstoff (*) in Verbindungen der Formel I,

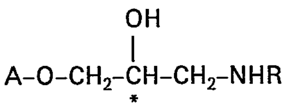

(I)

in der A für einen 2-Cyclopentyl-phenylrest und R für einen t-Butylrest steht, das dadurch gekennzeichnet ist, dass man Verbindungen der Formel I durch Formylierung in optisch aktive Verbindungen der Formel II

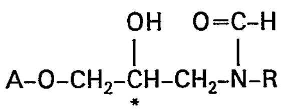

(II)

in der A und R die vorstehend definierten Bedeutungen haben, unter Konfigurationserhalt am Kohlenstoffatom (*) überführt, diese durch Behandeln mit einer starken Säure oder einem Säurehalogenid in optisch aktive cyclische Verbindungen der Formel III,

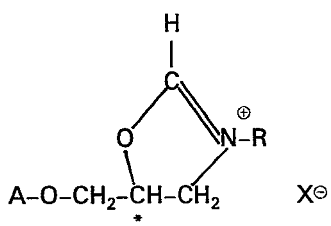

(III)

in der A und R die vorstehend definierten Bedeutungen haben und worin $X^{\ominus}$ für das Anion einer starken Säure oder eines Halogenatoms steht, überführt und diese Oxazoliniumderivate (III) durch saure oder alkalische Hydrolyse, gegebenenfalls über die Stufe der N-Formylverbindung in optisch aktive Verbindungen der Formel IV

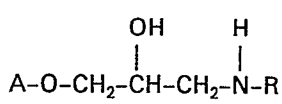

(IV)

in der A und R die vorstehend definierten Bedeutungen haben, mit der gleichen Konstitution wie das Ausgangsmaterial I, aber mit einer entgegengesetzten Konfiguration am Kohlenstoffatom (*) überführt, und wenn erwünscht, eine freie Verbindung der Formel I in ein Salz oder ein erhaltenes Salz in die freie Verbindung überführt.

Die erfindungsgemässe Konfigurationsumkehr bezieht sich auf die in der Gruppe

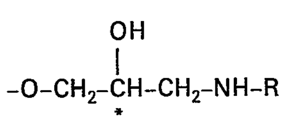

vorhandene sekundäre Alkoholgruppe, die ein Chiralitätszentrum enthält und die daher entweder in der S- oder in der R-Konfiguration vorliegen kann.

Dementsprechend wird nach dem erfindungsgemässen Verfahren ein (2S)-3-Amino-2-propanol der Formel I in das entsprechende (2R)-3-Amino-2-propanol bzw. ein (2R)-3-Amino-2-propanol der Formel I in das entsprechende (2S)-3-Amino-2-propanol umgewandelt. Liegt in einer Verbindung der Formel I kein weiteres Chiralitätszentrum vor, dann wird durch diese Konfigurationsumkehr eine Umkehrung des optischen Drehsinns bewirkt.

Rest R ist der tert.-Butylrest.

Aus der EP-A1 0 007 605 ist ein Verfahren zur Konfigurationsumkehr optisch aktiver Verbindungen bekannt. Bei diesem Verfahren wird eine optisch aktive Verbindung in das entsprechende N-Acylderivat der Formel V überführt.

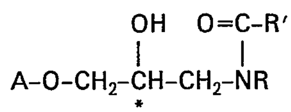

(V)

Bei diesem Verfahren ist R′ ein mono- oder polycyclischer, carbo- oder heterocyclischer Rest oder ein gegebenenfalls substituierter aliphatischer, cycloaliphatischer oder araliphatischer Kohlenwasserstoffrest.

Das N-Acylderivat (V) wird zum entsprechenden Oxazoliniumsalz der Formel VI

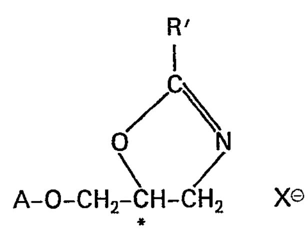

(VI)

cyclisiert, welches dann zu einer optisch aktiven Verbindung mit der gleichen Konstitution, aber der entgegengesetzten Konfiguration des Ausgangsmaterials hydrolysiert wird.

Bei Versuchen, dieses Verfahren auf die Umwandlung von 1-Aryloxy-3-tert.-butylamino-2-propanolen zu übertragen, wurde nun gefunden, dass derartige 1-Aryloxy-3-tert.-butylamino-2-propanole (R = tert.-Butyl) mit bekannten Acylierungsmitteln wie z.B. Essigsäure, Essigsäureanhydrid, Ethylacetat oder Acetylchlorid selbst in mehrtägiger Reaktion nur in Ausbeuten von weniger als 5% zu Verbindungen der Formel (V) (R′ = $CH_3$) überführt werden können. Daher konnten auch die für eine Konfigurationsumkehr nötigen cyclischen Zwischenprodukte (VI) (R′ = $CH_3$) nicht mehr in befriedigenden Ausbeuten hergestellt werden; eine verwertbare Konfigurationsumkehr liess sich so nicht erzielen.

Aus C.A. 84, Nr. 43 593x [1976] ist es bekannt, Aminoalkohole $RCH(OH)CHR^1NHR^2$ mit Hilfe von

Ameisensäureabkömmlingen zu acylieren und dann zu Oxazolidinonen zu cyclisieren, dann den Ring zwecks Umkehr der Konfiguration zu hydrolysieren oder zu reduzieren. Ein Hinweis auf die besondere Eignung der Formylierung für erfindungsgemäss substituierte Verbindungen findet sich jedoch nicht.

Überraschenderweise wurde nun gefunden, dass derartige 1-Aryloxy-3-tert.-butylamino-2-propanole der Formel I nach an sich bekannten Methoden mit Formylierungsmitteln in guten Ausbeuten in die entsprechenden N-Formylverbindungen der Formel II überführt werden können, die dann entweder in der Schmelze oder in einem geeigneten Lösungsmittel mit einer starken Säure oder mit einem Säurehalogenid bei Temperaturen von −20°C bis +150°C, vorzugsweise bei 0° bis 50°C zu den entsprechenden Oxazolinium-Salzen der Formel III cyclisiert werden.

Diese werden in saurem oder basischem Medium, gegebenenfalls über die Stufe der invertierten N-Formylverbindung, zu einem optisch aktiven 1-Aryloxy-3-tert.-butylamino-2-propanol der Formel (IV) mit der gleichen Konstitution, aber der entgegengesetzten Konfiguration des Ausgangsmaterials der Formel I hydrolysiert.

Ein weiterer Vorteil des neuen Verfahrens besteht in der Verwendung von Ameisensäure oder ihren Derivaten als Reagenz für die Herstellung der Zwischenprodukte, da Ameisensäure und ihre Derivate leicht verfügbar und (auf Stoffmengenbasis) im allgemeinen billiger sind als die homologen Carbonsäuren und ihre entsprechenden Derivate.

Als Formylierungsmittel sind beispielsweise Ameisensäure oder deren Derivate (z.B. gemischtes Anhydrid aus Ameisensäure und Acetanhydrid, Phenylformiat, Methylformiat, Ethylformiat und Butylformiat) geeignet. Anstelle der reinen Ameisensäureester können diese auch durch azeotrope Veresterung in situ hergestellt und ohne weitere Reinigung für die Formylierung der Amino-2-propanole der Formel I eingesetzt werden.

Als Cyclisierungsreagenzien kommen starke, sauerstoffhaltige anorganische oder organische Säuren wie z.B. konzentrierte Schwefelsäure oder Phosphorsäure oder eine starke organische Sulfonsäure, etwa eine aliphatische Sulfonsäure, z.B. Methansulfonsäure, oder eine aromatische Sulfonsäure, wie eine gegebenenfalls substituierte Phenylsulfonsäure oder deren Halogenide, in erster Linie die Chloride oder Bromide wie Thionylchlorid, Thionylbromid, Sulfurylchlorid, Chlorsulfonsäure, Phosphortrichlorid, Phosphorpentachlorid, Phosphoroxychlorid oder Methansulfonylchlorid in Betracht. Ferner können auch den genannten Halogeniden entsprechende gemischte Ester, wie etwa ein Niederalkoxysulfonylhalogenid oder Phosphorsäureniederalkylesterhalogenide verwendet werden. Die Cyclisierung wird entweder in der Schmelze oder in einem geeigneten inerten Lösungsmittel (z.B. Methylenchlorid, Toluol) durchgeführt.

Bei Verwendung von Säuren wird ein Temperaturbereich von 50° bis 150°C bevorzugt, während bei Verwendung eines Säurechlorids vorzugsweise bei −20° bis 80°C gearbeitet wird.

Die Hydrolyse wird in saurem oder basischem Medium durchgeführt. Geeignete saure Mittel sind dabei wässrige Säuren, etwa wässrige Mineralsäuren, z.B. wässrige Salzsäure, Schwefelsäure oder Phosphorsäure. Die saure Hydrolyse wird in einem Temperaturbereich von 0° bis +120°C, zweckmässigerweise bei +10 bis +50°C durchgeführt. Als basische Medien sind z.B. wässrige Laugen, etwa die der Alkalien oder Erdalkalien wie Natriumhydroxid, oder Kaliumhydroxid, oder die Hydroxide des Calciums oder Magnesiums geeignet, wobei die genannten Reagentien vorteilhafterweise bei erhöhter Temperatur, etwa in einem Bereich von 50 bis 150°C eingesetzt werden. Die Hydrolyse kann sowohl in homogener Phase als auch in einem Mehrphasensystem durchgeführt werden.

Das erfindungsgemässe Verfahren kann auch ohne Isolierung des Zwischenproduktes der Formel II durchgeführt werden, das dann im gleichen Reaktionsansatz zu der Verbindung der Formel III weiter verarbeitet und die erhaltene Verbindung der Formel III ohne weitere Reinigung der Hydrolyse unterworfen wird.

Die Erfindung betrifft ferner optisch aktive Verbindungen der Formel III, worin A, R und $X^{\ominus}$ obige Bedeutungen haben, oder solche Verbindungen als freie Basen.

Bevorzugt sind solche Verbindungen der Formel III, in der A und R wie vorstehend definiert sind und $X^{\ominus}$ für ein Anion der Schwefelsäure, Phosphorsäure oder einer starken organischen Sulfonsäure, wie p-Toluolsulfonsäure oder Benzolsulfonsäure, oder für Chlorid oder Bromid steht.

Die Verbindungen der Formel IV können aufgrund ihrer pharmakologischen Eigenschaften als Arzneimittel verwendet werden. Die folgenden Beispiele dienen zur Erläuterung der Erfindung, ohne diese auf die stellvertretend genannten Verbindungen zu beschränken:

Beispiel 1:

Inversion von (+)-1-(2-Cyclopentyl-phenoxy)-3-tert.-butylamino-2-propanol

a) (+)-1-(2-Cyclopentyl-phenoxy)-3-N-tert.-butyl-N-formylamino-2-propanol

Man erhitzt eine Lösung von 29,1 g 1-(2-Cyclopentyl-phenoxy)-3-tert.-butylamino-2-propanol (81% (+)- und 19% (−)-Konfiguration, $[\alpha]_D^{20}$ + 10.0° (5%ig in Isopropanol) in 60 g Ameisensäuremethylester während 48 Stunden am Rückfluss. Nach Eindampfen der Lösung im Vakuum erhält man das rohe (+)-1-(2-Cyclopentylphenoxy)-3-N-tert.-butyl-N-formylamino-2-propanol. Dieses kann aus n-Hexan umkristallisiert werden ($[\alpha]_D^{20}$ +11.0°).

b) 1-tert.-Butyl-4-(2-cyclopentyl-phenoxymethyl)-oxazoliniumchlorid

Das rohe (+)-1-(2-Cyclopentyl-phenoxy)-3-N-tert.-butyl-N-formylamino-2-propanol wird in

50 ml Toluol gelöst. Zu dieser Lösung werden unter Kühlen mit Eis 7,5 ml Thionylchlorid so zugetropft, dass die Temperatur 20°C nicht überschreitet. Man rührt 15 Minuten nach und erhält nach Eindampfen der Lösung im Vakuum das rohe 1-tert.-Butyl-3-(2-cyclopentyl-phenoxymethyl)-oxazoliniumchlorid.

Das rohe Oxazoliniumsalz kann aus Toluol umkristallisiert werden $[\alpha]_D^{20}$ +8.2°).

c) (−)-1-(2-Cyclopentyl-phenoxy)-3-tert.-butylamino-2-propanol

Das rohe 1-tert.-Butyl-4-(2-cyclopentylphenoxymethyl)-oxazoliniumchlorid wird in 60 ml Isopropanol gelöst, mit 400 ml 4-N-Natronlauge versetzt und 3 h unter intensivem Rühren am Rückfluss gekocht. Nach Abkühlen auf Raumtemperatur wird die organische Phase abgetrennt.

Sie enthält 27,0 g 1-(2-Cyclopentylphenoxy)-3-tert.-butylamino-2-propanol (67% (−)- und 33% (+)-Konfiguration, $[\alpha]_D^{20}$ = −5.5°C (gemessen in 5%iger isopropanolischer Lösung; Gehalt an Base titrimetrisch mit Salzsäure bestimmt). Dies entspricht einer optischen Ausbeute von 55%. Die isopropanolische Lösung wird filtriert und mit 12,2 g D-(−)-Mandelsäure versetzt, worauf das (−)-1-(2-Cyclopentylphenoxy)-3-tert.-butylamino-2-propanol-D-(−)-mandelat ausfällt, welches durch Umkristallisieren aus 50 ml Isopropanol gereinigt wird.

Ausbeute: 22.0 g ($[\alpha]_D^{20}$ = −52.4° (1%ig in Isopropanol)) ≙ 61.3% der im Ausgangsmaterial enthaltenen (+)-Base ≙ 74.1% der in der Verseifungslösung enthaltenen (−)-Base.

Beispiel 2:

Inversion von (+)-1-(2-Cyclopentyl-phenoxy)-3-tert.-butylamino-2-propanol

a) (+)-1-(2-Cyclopentyl-phenoxy)-3-N-tert.-butyl-N-formylamino-2-propanol

291,0 g 1-(2-Cyclopentyl-phenoxy)-3-tert.-butylamino-2-propanol (81% (+)- und 19% (−)-Konfiguration, $[\alpha]_D^{20}$ = +10.0° (5%ig in Isopropanol) und 306 g Ameisensäurebutylester werden 6 Stunden zum Rückfluss erhitzt. Nach Eindampfen des Reaktionsgemisches im Vakuum erhält man das rohe (+)-1-(2-Cyclopentyl-phenoxy)-3-N-tert.-butylamino-N-formylamino-2-propanol.

b) 1-tert.-Butyl-4-(2-cyclopentyl-phenoxymethyl)-oxazoliniumchlorid

Das rohe (+)-1-(2-Cyclopentyl-phenoxy)-3-N-tert.-butyl-N-formylamino-2-propanol wird in 240 ml Toluol gelöst. Zu dieser Lösung werden unter Kühlung 95 ml Thionylchlorid so zugetropft, dass die Temperatur 40°C nicht überschreitet. Bei dieser Temperatur wird 2 Stunden nachgerührt.

c) (−)-1-(2-Cyclopentyl-phenoxy)-3-tert.-butylamino-2-propanol

Die toluolische Lösung des rohen 1-tert.-Butyl-4-(2-cyclopentylphenoxymethyl)--oxazoliniumchlorids wird sauer verseift, indem man diese Lösung zu 100 ml Wasser so zutropft, dass die Temperatur 60°C nicht überschreitet. Es wird 2 Stunden bei 60°C nachgerührt und sodann mit 800 ml 18%iger Natronlauge versetzt. Die toluolische Phase wird abgetrennt, mit 100 ml Wasser gewaschen und filtriert; sie enthält 280,2 g (−)-1-(2-Cyclopentyl-phenoxy)-3-tert.-butylamino-2-propanol (Titration mit Salzsäure).

$[\alpha]_D^{20}$: −8.2° (5%ig in Isopropanol; 76% (−)- und 24% (+)-Konfiguration)

opt. Ausbeute: 82%

Die weitere Reinigung erfolgt über das diastereomere Salz mit D-(−)-Mandelsäure, wie in Beispiel 1c) beschrieben.

Beispiel 3

Inversion von (+)-1-(2-Cyclopentyl-phenoxy)-3-tert.-butylamino-2-propanol

a) (+)-1-(2-Cyclopentyl-phenoxy)-3-N-tert.-butyl-N-formylamino-2-propanol

Man erhitzt eine Mischung von 110 g Ameisensäure 85%ig, 148 g n-Butanol und 35 g Toluol am Wasserabscheider. Nach 2 h haben sich 50 ml Wasser (mit 11% Ameisensäure) abgeschieden, das Destillat läuft klar ab. Zu dieser Lösung werden 291 g 1-(2-Cyclopentyl-phenoxy)-3-tert.-butylamino-2-propanol (81% (+)- und 19% (−)-Konfiguration, $[\alpha]_D^{20}$ + 10.0° (5%ig in Isopropanol)) gegeben und 16 Stunden am Rückfluss erhitzt. Die Lösungsmittel (Butylformiat, Butanol, Toluol) werden zunächst bei Normaldruck, dann im Vakuum abgezogen; man erhält das rohe (+)-1-(2-Cyclopentyl-phenoxy)-3-N-tert.-butyl-N-formylamino-2-propanol.

Das abgezogene Lösungsmittelgemisch kann für weitere gleichartige Reaktionen nach Ergänzen der Ameisensäure wiederverwendet werden.

Die weitere Aufarbeitung erfolgt wie in Beispiel 2b) bzw. 2c) beschrieben. Die toluolische Lösung enthält

271.2 g (−)-1-(2-Cyclopentyl-phenoxy)-3-tert.-butylamino-2-propanol (Titration mit Salzsäure).

$[\alpha]_D^{20}$: −7,2° (5%ig in Isopropanol; 73% (−)- und 27% (+)-Konfiguration).

opt. Ausbeute: 72%

Beispiel 4:

Inversion von (+)-1-(2-Cyclopentyl-phenoxy)-3-tert.-butylamino-2-propanol

a) (+)-1-(2-Cyclopentyl-phenoxy)-3-N-tert.-butyl-N-formylamino-2-propanol

145,5 g 1-(2-Cyclopentyl-phenoxy)-3-tert.-butylamino-2-propanol (81% (+)- und 19% (−)-Konfiguration, $[\alpha]_D^{20}$ = 10.0° (5%ig in Isopropanol)) werden in 172 ml Toluol gelöst und 54,1 g Ameisensäure 85%ig bei Raumtemperatur zugetropft. Man erhitzt ca. 7 Stunden am Wasserabscheider, wobei sich 31 g Wasser (mit ca. 60% Ameisensäure) abscheiden. Nach Abdestillieren des Lösungsmittels und überschüssiger Ameisensäure im Vakuum verbleibt das rohe (+)-1-(2-Cyclopentyl-phenoxy)-3-N-tert.-butyl-N-formylamino-2-propanol als Rückstand.

b) 1-tert.-Butyl-4-(2-cyclopentyl-phenoxymethyl)-oxazoliniumchlorid

Zu einer Lösung des rohen (+)-1-(2-Cyclopentyl-phenoxy)-3-N-tert.-butyl-N-formylamino-2-propanols in 150 ml Toluol werden unter Kühlung 37,5 ml Thionylchlorid so zugetropft, dass eine Temperatur von 40°C nicht überschritten wird. Anschliessend wird bei dieser Temperatur 2 Stunden nachgerührt.

c) (−)-1-(2-Cyclopentyl-phenoxy)-3-tert.-butylamino-2-propanol

Die toluolische Lösung des rohen 1-tert.-Butyl-3-(2-cyclopentylphenoxymethyl)--oxazoliniumchlorids wird unter Rühren zu 50 ml Wasser so zugetropft, dass eine Temperatur von 60°C nicht überschritten wird. Man rührt weitere 2 Stunden bei 60°C und versetzt die Lösung anschliessend mit 375 ml 18%iger Natronlauge. Die organische Phase wird abgetrennt, mit 50 ml Wasser gewaschen und filtriert; sie enthält 130,9 g (−)-1-(2-Cyclopentyl-phenoxy)-3-tert.-butylamino-2-propanol (Titration mit Salzsäure). $[\alpha]_D^{20}$: −5,7° (5%ig in Isopropanol; 68% (−)- und 32% (+)-Konfiguration)
opt. Ausbeute: 57%

Die weitere Reinigung erfolgt über das diastereomere Salz mit D-(−)-Mandelsäure, wie in Beispiel 1c) beschrieben.

**Patentansprüche für die Vertragsstaaten BE, CH, DE, FR, GB, IT, LI, NL, SE**

1. Verfahren zur Umkehr der Konfiguration am optisch aktiven Kohlenstoffatom (*) in Verbindungen der Formel I,

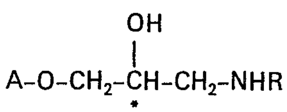
(I)

in welcher A einen 2-Cyclopentylphenylrest und R den t-Butylrest bedeuten, dadurch gekennzeichnet, dass man diese durch Formylierung in optisch aktive Verbindungen der Formel II

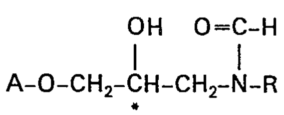
(II)

in welcher A und R die vorstehend definierten Bedeutungen haben, unter Konfigurationserhalt am Kohlenstoffatom (*) überführt, diese durch Behandeln mit einer starken Säure oder einem Säurehalogenid in optisch aktive cyclische Verbindungen der Formel III

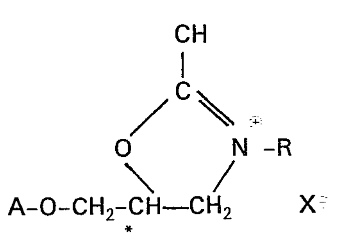
(III)

in welcher A und R die vorstehend definierten Bedeutungen haben und $X^-$ für das Anion einer starken Säure oder eines Halogenatoms steht, überführt und diese Oxazoliniumderivate (III) durch saure oder alkalische Hydrolyse, gegebenenfalls über die Stufe der N-Formylverbindung in optisch aktive Verbindungen der Formel IV

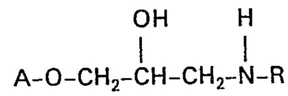
(IV)

in welcher A und R die vorstehend definierten Bedeutungen haben mit der gleichen Konstitution wie das Ausgangsmaterial I, aber der entgegengesetzten Konfiguration am Kohlenstoffatom (*) überführt.

2. Verfahren nach Anspruch 1, dadurch gekennzeichnet, dass man als Säurehalogenid Sulfurylchlorid, Thionylbromid, Thionylchlorid, Phosphortrichlorid, Phosphoroxychlorid, Phosphorpentachlorid, Toluolsulfonsäurechlorid oder Methansulfonsäurechlorid verwendet.

3. Verfahren nach Anspruch 1, dadurch gekennzeichnet, dass man unter Verwendung einer starken Säure im Temperaturbereich von +50°C bis 150°C cyclisiert.

4. Verfahren nach Anspruch 1, dadurch gekennzeichnet, dass man unter Verwendung eines Säurehalogenids im Temperaturbereich von −20° bis 80°C cyclisiert.

5. Verbindung der Formel III, in welcher A, R und $X^\ominus$ die in Anspruch 1 definierten Bedeutungen haben oder die entsprechende freie Base.

6. Verbindung der Formel III gemäss Anspruch 5, dadurch gekennzeichnet, dass $X^{(-)}$ ein Anion der Schwefelsäure, Phosphorsäure oder einer starken organischen Sulfonsäure, Chlorid oder Bromid bedeutet.

**Patentansprüche für den Vertragsstaat AT**

1. Verfahren zur Umkehr der Konfiguration am optisch aktiven Kohlenstoffatom (*) in Verbindungen der Formel I,

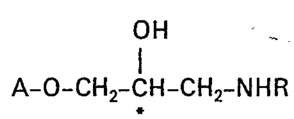
(I)

in welcher A einen 2-Cyclopentylphenylrest und R den t-Butylrest bedeuten, dadurch gekennzeichnet, dass man diese durch Formylierung in optisch aktive Verbindungen der Formel II

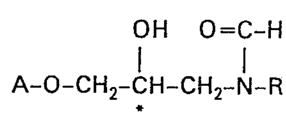
(II)

in welcher A und R die vorstehend definierten Bedeutungen haben, unter Konfigurationserhalt am Kohlenstoffatom (*) überführt, diese durch Behandeln mit einer starken Säure oder einem Säurehalogenid in optisch aktive cyclische Verbindungen der Formel III

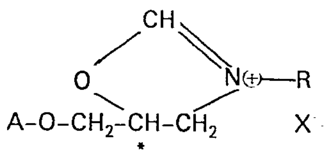

(III)

in welcher A und R die vorstehend definierten Bedeutungen haben und $X^-$ für das Anion einer starken Säure oder eines Halogenatoms steht, überführt und diese Oxazoliniumderivate (III) durch saure oder alkalische Hydrolyse, gegebenenfalls über die Stufe der N-Formylverbindung in optisch aktive Verbindungen der Formel IV

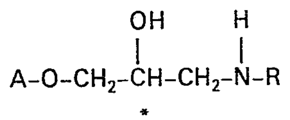

(IV)

in welcher A und R die vorstehend definierten Bedeutungen haben mit der gleichen Konstitution wie das Ausgangsmaterial I, aber der entgegengesetzten Konfiguration am Kohlenstoffatom (*) überführt.

2. Verfahren nach Anspruch 1, dadurch gekennzeichnet, dass man als Säurehalogenid Sulfurylchlorid, Thionylbromid, Thionylchlorid, Phosphortrichlorid, Phosphoroxychlorid, Phosphorpentachlorid, Toluolsulfonsäurechlorid oder Methansulfonsäurechlorid verwendet.

3. Verfahren nach Anspruch 1, dadurch gekennzeichnet, dass man unter Verwendung einer starken Säure im Temperaturbereich von +50°C bis 150°C cyclisiert.

4. Verfahren nach Anspruch 1, dadurch gekennzeichnet, dass man unter Verwendung eines Säurehalogenids im Temperaturbereich von −20° bis 80°C cyclisiert.

5. Verfahren zur Herstellung von Verbindungen der Formel III

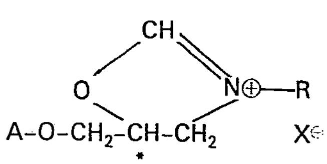

(III)

in welcher A, R und $X^\ominus$ die in Anspruch 1 definierten Bedeutungen haben oder von entsprechenden freien Basen, dadurch gekennzeichnet, dass man Verbindungen der Formel I

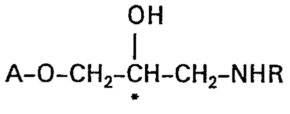

(I)

durch Formylierung in optisch aktive Verbindungen der Formel II

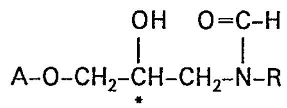

(II)

worin A und R die im Anspruch 1 angegebenen Bedeutungen haben, überführt und diese durch Behandeln mit einer starken Säure oder einen Säurehalogenid in optisch aktiv cyclische Verbindungen der Formel III überführt.

6. Verfahren gemäss Anspruch 5, dadurch gekennzeichnet, dass $X^{(-)}$ ein Anion der Schwefelsäure, Phosphorsäure oder einer starken organischen Sulfonsäure, Chlorid oder Bromid bedeutet.

**Claims for the contracting states BE, CH, DE, FR, GB, IT, LI, NL, SE**

1. A process for inverting the configuration at the optically active carbon atom (*) in compounds of the formula I

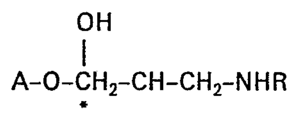

(I)

in which A represents a cyclopentylphenyl radical and R represents the t-butyl radical, which comprises converting these compounds by formylation into optically active compounds of the formula II

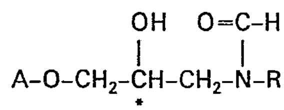

(II)

in which A and R have the meanings defined above, while maintaining the configuration at the carbon atom (*), converting these compounds, by treatment with a strong acid or an acid halide, into optically active cyclic compounds of the formula III

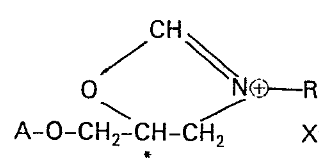

(III)

in which A and R have the meanings defined above and in which $X^\ominus$ represents the anion of a strong acid or of a halogen atom, and converting these oxazolinium derivatives (III), by acid or alkaline hydrolysis, if appropriate via the stage of the N-formyl compound, into optically active compounds of the formula IV

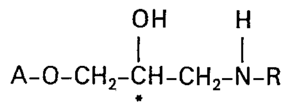

in which A and R have the meanings defined above, possessing the same structure as the starting material I, but having an opposite configuration at the carbon atom (*).

2. The process as claimed in claim 1, wherein the acid halide used is sulfuryl chloride, thionyl bromide, thionyl chloride, phosphorus trichloride, phosphorus oxychloride, phosphorus pentachloride, toluenesulfonyl chloride or methanesulfonyl chloride.

3. The process as claimed in claim 1, wherein cyclization is carried out within the temperature range from +50°C to 150°C using a strong acid.

4. The process as claimed in claim 1, wherein cyclization is carried out within the temperature range from −20°C to 80°C using an acid halide.

5. A compound of the formula III in which A, R and X⊖ have the meanings defined in claim 1, or the corresponding free base.

6. A compound of the formula III as claimed in claim 5, wherein X⊖ denotes an anion of sulfuric acid, phosphoric acid or a strong organic sulfuric acid, or a chloride or bromide.

**Claims for the contracting state AT**

1. A process for inverting the configuration at the optically active carbon atom (*) in compounds of the formula I

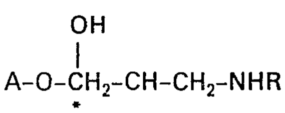

(I)

in which A represents a cyclopentylphenyl radical and R represents the t-butyl radical, which comprises converting these compounds by formylation into optically active compounds of the formula II

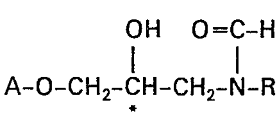

(II)

in which A and R have the meanings defined above, while maintaining the configuration at the carbon atom (*), converting these compounds, by treatment with a strong acid or an acid halide, into optially active cyclic compounds of the formula III

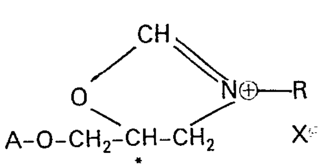

(III)

in which A and R have the meanings defined above and in which X⊖ represents the anion of a strong acid or of a halogen atom, and converting these oxazolinium derivatives (III), by acid or alkaline hydrolysis, if appropriate via the stage of the N-formyl compound, into optically active compounds of the formula IV

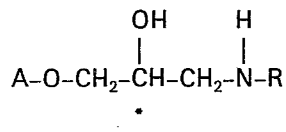

(IV)

in which A and R have the meanings defined above, possessing the same structure as the starting material I, but having an opposite configuration at the carbon atom (*).

2. The process as claimed in claim 1, wherein the acid halide used is sulfuryl chloride, thionyl bromide, thionyl chloride, phosphorus trichloride, phosphorus oxychloride, phosphorus pentachloride, toluenesulfonyl chloride or methanesulfonyl chloride.

3. The process as claimed in claim 1, wherein cyclization is carried out within the temperature range from +50°C to 150°C using a strong acid.

4. The process as claimed in claim 1, wherein cyclization is carried out within the temperature range from −20°C to 80°C using an acid halide.

5. A process for the production of compounds of the formula III,

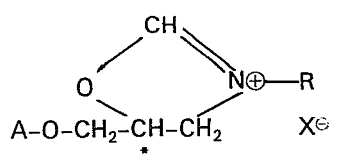

(III)

in which A, R and X⊖ have the meanings defined in claim 1, or the corresponding free base, characterized in that compounds of the formula I

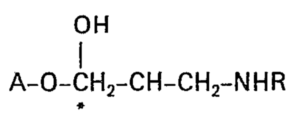

(I)

are converted by formylation into optically active compounds of the formula II

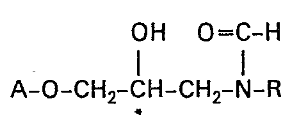

(II)

in which A and R have the meanings defined in claim 1, and converting these compounds, by treatment with a strong acid or an acid halide, into optically active cyclic compounds of the formula III.

6. A process as claimed in claim 5, wherein X⊖ denotes an anion of sulfuric acid, phosphoric acid or a strong organic sulfuric acid or a chloride or bromide.

**Revendications pour les Etats contractants BE, CH, DE, FR, GB, IT, LI, NL, SE**

1. Procédé pour l'inversion de la configuration au niveau de l'atome de carbone optiquement actif (*) dans des composés de formule I

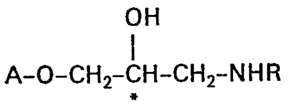

(I)

dans laquelle A représente un reste 2-cyclopentyl-phényle et R le reste tert-butyle, caractérisé en ce que l'on transforme ceux-ci par formylation en composés optiquement actifs de formule II

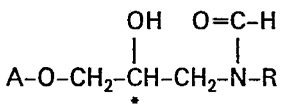

(II)

dans laquelle A et R ont les significations définies précédemment, avec rétention de la configuration au niveau de l'atome de carbone (*), on transforme ceux-ci, par traitement avec un acide fort ou un halogénure d'acide, en composés cycliques optiquement actifs de formule III

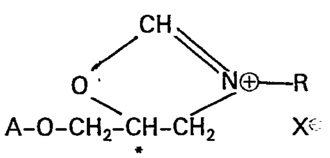

(III)

dans laquelle A et R ont les significations définies précédemment et X⊖ représente l'anion d'un acide fort ou d'un atome d'halogène, puis on transforme ces dérivés d'oxazolinium (III), par hydrolyse acide ou alcaline, éventuellement en passant par le stade du composé N-formyle, en composés optiquement actifs de formule IV

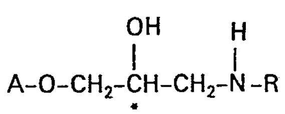

(IV)

dans laquelle A et R ont les significations définies précédemment, qui ont la même constitution que le produit de départ I, mais la configuration opposée au niveau de l'atome de carbone (*).

2. Procédé selon la revendication 1, caractérisé en ce que l'on utilise comme halogénure d'acide le chlorure de sulfuryle, le bromure de thionyle, le chlorure de thionyle, le trichlorure de phosphore, l'oxychlorure de phosphore, le pentachlorure de phosphore, le chlorure d'acide toluènesulfonique ou le chlorure d'acide méthanesulfonique.

3. Procédé selon la revendication 1, caractérisé en ce que l'on effectue la cyclisation en utilisant un acide fort, à une température allant de +50 à 150°C.

4. Procédé selon la revendication 1, caractérisé en ce que l'on effectue la cyclisation en utilisant un halogénure d'acide à une température allant de −20 à 80°C.

5. Composé de formule III, dans lequel A, R et X⊖ ont les significations définies dans la revendication 1, ou la base libre correspondante.

6. Composé de formule III selon la revendication 5, caractérisé en ce que X⊖ représente un anion de l'acide sulfurique, de l'acide phosphorique ou d'un acide sulfonique organique fort, l'anion chlorure ou bromure.

**Revendications pour l'Etat contractant AT**

1. Procédé pour l'inversion de la configuration au niveau de l'atome de carbone optiquement actif (*) dans des composés de formule I

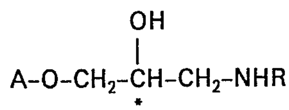

(I)

dans laquelle A représente un reste 2-cyclopentyl-phényle et R le reste tert-butyle, caractérisé en ce que l'on transforme ceux-ci par formylation en composés optiquement actifs de formule II

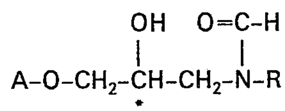

(II)

dans laquelle A et R ont les significations définies précédemment, avec rétention de la configuration au niveau de l'atome de carbone (*), on transforme ceux-ci, par traitement avec un acide fort ou un halogénure d'acide, en composés cycliques optiquement actifs de formule III

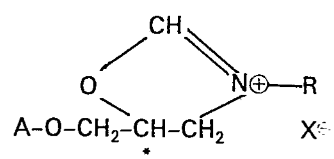

(III)

dans laquelle A et R ont les significations définies précédemment et X⊖ représente l'anion d'un acide fort ou d'un atome d'halogène, puis on transforme ces dérivés d'oxazolinium (III), par hydrolyse acide ou alcaline, éventuellement en passant par le stade du composé N-formyle, en composés optiquement actifs de formule IV

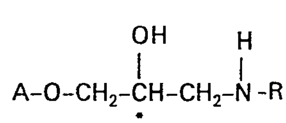

(IV)

dans laquelle A et R ont les significations définies précédemment, qui ont la même constitution que le produit de départ I, mais la configuration opposée au niveau de l'atome de carbone (*).

2. Procédé selon la revendication 1, caractérisé en ce que l'on utilise comme halogénure d'acide le chlorure de sulfuryle, le bromure de thionyle, le chlorure de thionyle, le trichlorure de phosphore, l'oxychlorure de phosphore, le pentachlorure de phosphore, le chlorure d'acide toluènesulfonique ou le chlorure d'acide méthanesulfonique.

3. Procédé selon la revendication 1, caractérisé en ce que l'on effectue la cyclisation en utilisant un acide fort, à une température allant de +50 à 150°C.

4. Procédé selon la revendication 1, caractérisé en ce que l'on effectue la cyclisation en utilisant un halogénure d'acide à une température allant de −20 à 80°C.

5. Procédé pour la préparation de composés de formule III

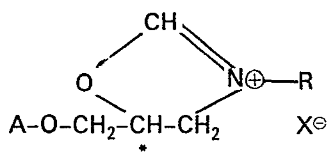

(III)

dans laquelle A, R et X⊖ ont les significations définies dans la revendication 1, ou de bases libres correspondantes, caractérisé en ce que l'on transforme des composés de formule I

$$A\text{-}O\text{-}CH_2\text{-}\overset{\displaystyle OH}{\underset{*}{\overset{|}{C}}}H\text{-}CH_2\text{-}NHR \qquad (I)$$

par formylation, en composés optiquement actifs de formule II

$$A\text{-}O\text{-}CH_2\text{-}\overset{\displaystyle OH}{\underset{*}{\overset{|}{C}}}H\text{-}CH_2\text{-}\overset{\displaystyle O{=}C\text{-}H}{\overset{|}{N}}\text{-}R \qquad (II)$$

dans laquelle A et R ont les significations données dans la revendication 1, et on transforme ceux-ci, par traitement avec un acide fort ou un halogénure d'acide, en composés cycliques optiquement actifs de formule III.

6. Procédé selon la revendication 5, caractérisé en ce que X⊖ représente un anion de l'acide sulfurique, de l'acide phosphorique ou d'un acide sulfonique organique fort, l'anion chlorure ou bromure.